# EUROPÄISCHE PATENTSCHRIFT

(11) **EP 3 013 304 B1**
(45) Veröffentlichungstag und Bekanntmachung des Hinweises auf die Patenterteilung: **11.12.2019**
(21) Anmeldenummer: 14801943.3
(22) Anmeldetag: 22.10.2014
(51) Int. Cl.: A61J 1/14, A61J 1/10

(54) **BEHAELTNIS UND SET ZUR BEREITSTELLUNG PARENTERALER NAHRUNG**
CONTAINER AND KIT FOR PROVIDING PARENTERAL NUTRITION
CONTENANT ET KIT DE NUTRITION PARENTÉRALE

(30) Priorität: 07.02.2014 DE 102014202261
(43) Veröffentlichungstag der Anmeldung: 04.05.2016
(60) Teilanmeldung: 19172546.4
(73) Patentinhaber: Eurozyto GmbH, 61462 Königstein (DE)
(72) Erfinder: ROSE, Uwe-Bernd, 61462 Königstein (DE)
(74) Vertreter: Patent- und Rechtsanwälte Ullrich & Naumann
(86) Internationale Anmeldenummer: PCT/EP2014/072646
(87) Internationale Veröffentlichungsnummer: WO 2015/117686

(56) Entgegenhaltungen:
- EP-A2- 0 132 632
- WO-A1-98/10733
- WO-A1-2007/016615
- WO-A1-2012/076690
- AU-B2- 674 028
- FR-A1- 2 645 437
- US-A1- 2002 138 066
- US-A1- 2013 108 189

## Beschreibung

Die Erfindung betrifft ein Behältnis, im Konkreten einen Beutel, zur Bereitstellung parenteraler Nahrung, mit einem in getrennte Kammern unterteilen Innenraum, wobei die Kammern zur Aufnahme von Flüssigkeiten zur parenteralen Ernährung dienen.

In der Medizin wird zwischen einer enteralen und einer parenteralen Ernährung unterschieden. Parenterale Ernährung ist eine Form der künstlichen Ernährung, bei der - bedingt durch eine akute Erkrankung von Magen und/oder Darm - der Magen-Darm-Trakt umgangen wird. Die Ernährung erfolgt dabei regelmäßig über spezielle Lösungen, die intravenös in eine der großen Körpervenen infundiert werden. Daher unterscheiden sich die zur parenteralen Ernährung eingesetzten Lösungen in ihrer Zusammensetzung von enteralen Ernährungslösungen.

Die Verabreichung parenteraler Lösungen kann auf verschiedene Arten erfolgen.

Eine erste Möglichkeit besteht in der Verordnung industriell vorgefertigter Mehrkammerbeutel, in denen die Ernährungskomponenten voneinander getrennt in Kammern gefüllt sind. Durch Auflösen oder Zerstören der Kammerwandungen kurz vor der Applikation werden die Komponenten miteinander vermischt. Derartige Fertigbeutel weisen eine Arzneimittelzulassung auf und sind in ihrer Zusammensetzung stets gleich. Ein Eingehen auf die individuelle Stoffwechsellage eines Patienten ist dadurch nicht in hinreichendem Maße möglich. Zusätzlich benötigte Nahrungskomponenten oder Arzneimittel müssen einzeln zugespritzt werden.

Alternativ hierzu kann der Patient entsprechend seiner Stoffwechsellage eine individuelle Ernährungslösung in einer Apotheke oder in einem dafür zugelassenen Herstellungsbetrieb nach § 13 AMG (Arzneimittelgesetz) zubereitet bekommen. Hierfür stehen sterile Behältnisse zur Verfügung, die eine bis maximal drei Kammern aufweisen. Im Falle von Einkammerbeuteln reduziert sich die Haltbarkeit der Ernährungslösung auf wenige Tage. Außerdem besteht Kühlpflicht, was zu einer aufwändigen und sicherheitskritischen Handhabung seitens Pflegediensten und Patienten führt. Im Falle von Mehrkammerbeuteln mit drei Kammern besteht wie auch bei vorgefertigten Beuteln das Problem, dass zusätzlich benötigte Nahrungskomponenten oder Arzneimittel einzeln zugespritzt werden müssen.

Derartige Mehrkammerbeutel sind bspw. aus der EP 0 619 998 B1, der EP 0 893 982 B1 oder der EP 0 883 396 B1 bekannt.

Die WO 2012/076690 A1 zeigt Schließvorrichtungen für Infusionsbeutel mit mehreren Kammern, wobei die Kammern durch Öffnen der Schließvorrichtungen miteinander verbindbar sind.

Aus der EP 0 132 632 A2 ist ein Beutel zur Bereitstellung steriler Komponenten mit zwei Kammern bekannt, die über einen durch Klammern verschließbaren Kanal miteinander verbindbar sind.

Aus der US 2002/0138066 A1 ist ein Mehrfachkammerbeutel zur Bereitstellung einer Krankheitserreger abtötenden Flüssigkeit bekannt. Der Beutel weist einen durch eine Klemmschiene in mehrere getrennte Kammern unterteilten Innenraum auf, wobei durch Herausziehen einer Litze eine Flüssigkeitsverbindung herstellbar ist.

Die US 2013/0108189 A1 zeigt ein Verfahren zur Herstellung von sterilen und flexiblen, mit einem Produkt gefüllten Beuteln aus einer gefalteten Folienbahn und entsprechende Beutel.

Aus der AU 674 028 B2 ist ein aus einer Folienbahn durch eine Hochfrequenzschweißelektrode hergestellten Mehrfachkammerbeutel zur medizinischen Perfusion bekannt, wobei die Kammern durch Klemmschienen gebildet sind und durch Herausziehen einer Litze eine Flüssigkeitsverbindung zwischen den Kammern herstellbar.

Die FR 2 645 437 A1 zeigt eine Vorrichtung zum Aufteilen eines flexiblen Beutels zur Bereitstellung von Medikamenten oder Lebensmitteln in unterschiedliche Kammern.

Aus der WO 2007/016615 A1 ist ein sterilisierter Mehrkammerbeutel für die parenterale Ernährung bekannt.

Der vorliegenden Erfindung liegt daher die Aufgabe zugrunde, ein Behältnis sowie ein Set der eingangs genannten Art derart auszugestalten und weiterzubilden, dass bei einfacher Handhabung eine Verabreichung parenteraler Nahrung entsprechend der individuellen Stoffwechsellage eines Patienten ermöglicht ist.

Erfindungsgemäß wird die voranstehende Aufgabe durch einen Beutel mit den Merkmalen des Anspruchs 1 gelöst. Danach ist der in Rede stehende Beutel derart ausgestaltet und weitergebildet, dass der Innenraum in getrennte Kammern unterteilt ist, wobei die Kammern zur Aufnahme von Flüssigkeiten zur parenteralen Ernährung dienen, wobei im Innenraum mindestens zwei Kammern ausgebildet sind, zwischen denen eine Flüssigkeitsverbindung herstellbar ist, wobei die Kammern durch sich quer zur Längsrichtung und über die gesamte Breite des Beutels erstreckende Klemmschienen gebildet und gegeneinander abgegrenzt sind. In den Klemmschienen sind Litzen angeordnet, die in der Klemmschiene klemmen und die ganz oder teilweise aus der Klemmschiene herausziehbar sind, wodurch die Flüssigkeitsverbindung zwischen den Kammern herstellbar ist, und wobei der Innenraum des Beutels in mindestens sechs und vorzugsweise maximal zehn, Kammern unterteilt ist. Mindestens zwei Kammern enthalten entsprechend der individuellen Stoffwechsellage eines Patienten unterschiedliche Ernährungskomponenten, die nach Herstellung der Flüssigkeitsverbindung zwischen den Kammern und vor Verabreichung an den Patienten zu einer parenteralen Nahrung vermischbar sind, wobei mindestens eine erste Kammer eine parenterale Nährlösung mit einem ersten Energiegehalt enthält und mindestens eine zweite Kammer eine Flüssigkeit zur Deckung des Flüssigkeitsbedarfs mit einem zweiten Energiegehalt enthält, der kleiner als der erste Energiegehalt der parenteralen Nährlösung ist, wobei die parenterale Nährlösung Kohlenhydrate, Aminosäuren, Fette, Vitamine, Spurenelemente und Elektrolyte umfasst.

In erfindungsgemäßer Weise ist zunächst erkannt worden, dass die Handhabung im Rahmen einer parenteralen Ernährung erheblich vereinfacht wird, wenn gleichzeitig mehrere Ernährungskomponenten, bspw. eine parenterale Ernährungslösung und eine Flüssigkeit zur Deckung des Flüssigkeitsbedarfs, bereitgestellt werden können. Damit lässt sich ein Grundbedarf der Ernährung auf einfache Weise decken.

Der Beutel der eingangs genannten Art ist derart ausgestaltet und weitergebildet, dass bei einfacher Handhabung einer Verabreichung parenteraler Nahrung entsprechend der individuellen Stoffwechsellage eines Patienten gewährleistet ist.

Vorzugsweise ist die Flüssigkeitsverbindung zwischen den Kammern regulierbar, wobei eine solche Regulierbarkeit als Ausführungsbeispiel eines erfindungsgemäßen Beutel zu verstehen ist.

Insoweit ist erkannt worden, dass es von besonderem Vorteil ist, wenn nicht nur eine stets gleiche Mischung der Flüssigkeitskomponenten, sondern durch Regulieren der Flüssigkeitsverbindung zwischen den Kammern eine individuelle Mischung entsprechend dem tatsächlichen Bedarf eines Patienten bereitgestellt werden kann. Ein Regulieren der Flüssigkeitsverbindung kann dabei durch Öffnen und Schließen der Flüssigkeitsverbindung, bspw. einem zwischen den Kammern ausgebildeten Kanal, oder auch durch ein teilweises Öffnen oder ein teilweises Schließen erfolgen. Mit anderen Worten ist der Öffnungsgrad der Flüssigkeitsverbindung oder der die Flüssigkeitsverbindung verwirklichenden Kanäle regulierbar. Auf Grund der durch die Regulierung möglichen Wiederverschließbarkeit einer Kammer lässt sich auch ein erneutes Befüllen einer Kammer realisieren. Auch eine intermittierende Zugabe einer Ernährungskomponente, bspw. im Wege einer nur intermittierend stattfindenden Flüssigkeitsverbindung, ist möglich.

Der Beutel kann als polymerer Mehrkammerbeutel ausgebildet sein. Der Beutel kann aus einer flexiblen Kunststofffolie bestehen, die doppelt gelegt, nämlich an einer Seite "umgeschlagen" ist. Durch eine Verschweißung können die offenen Seiten verschlossen werden. Als vorteilhafte Dimensionierung mit hinreichendem Volumen hat sich eine Beutelgröße mit einer Länge von 600 mm und einer Breite von 250 mm herausgestellt. Durch die Flexibilität des aus Kunststofffolie bestehenden Beutels kann dieser auch problemlos mobil eingesetzt werden, wenn die Patienten Wert auf Mobilität legen und die Entleerung des Beutels über eine mobile Pumpe erfolgt.

Erfindungsgemäß enthält mindestens eine erste Kammer eine parenterale Nährlösung mit einem ersten Energiegehalt und enthält mindestens eine zweite Kammer eine Flüssigkeit zur Deckung des Flüssigkeitsbedarfs mit einem zweiten Energiegehalt, der kleiner als der erste Energiegehalt ist. Damit ist der Beutel bereits gebrauchsfertig und eine Applikation der parenteralen Nahrung kann erfolgen. Im Konkreten kann die erste Kammer ein größeres Volumen als die zweite Kammer aufweisen. Somit ist ein größeres Volumen der parenteralen Nährlösung bereitgestellt. Hierdurch ist eine hinreichende Abstimmung der parenteralen Ernährung realisiert, so dass diese hinsichtlich des Energie- und Nährstoffgehalts bedarfsdeckend ist. Aufgrund der Tatsache, dass bereits die parenterale Nährlösung einen gewissen Flüssigkeitsgehalt aufweist, kann das Volumen der zweiten Kammer, beinhaltend die Flüssigkeit zur Deckung des Flüssigkeitsbedarfs, entsprechend geringer dimensioniert werden. Dabei ist denkbar, dass die erste Kammer 750 bis 1.000 ml parenterale Ernährungslösung enthält und/oder dass die zweite Kammer 250 bis 750 ml Flüssigkeit zur Deckung des Flüssigkeitsbedarfs enthält.

Für eine möglichst vielseitige Anpassbarkeit der parenteralen Ernährung an den Bedarf eines Patienten können im Innenraum weitere Kammern vorgesehen sein, die zur bedarfsgerechten Bereitstellung von Flüssigkeiten im Volumen unterschiedlich, insbesondere gestuft, dimensioniert sind. Somit können weitere Flüssigkeiten bereitgestellt werden, und zwar in Abhängigkeit vom konkreten oder möglichen Bedarf eines Patienten. In Abhängigkeit von den individuellen Bedürfnissen des Patienten werden dann nur einzelne oder alle der Kammern entleert oder miteinander gemischt, und zwar mittels der regulierbaren Flüssigkeitsverbindung zwischen den Kammern. In den weiteren Kammern können sich weitere energietragende, parenterale Ernährungskomponenten oder sonstige Vitamin- oder Mineralstoffkomponenten befinden. Somit ist die Bereitstellung einer bedarfsdeckenden Nahrung nochmals verbessert. Von ganz besonderem Vorteil ist dabei, dass die parenterale Ernährungslösung, die Flüssigkeit zur Deckung des Flüssigkeitsbedarfs sowie weitere Ernährungskomponenten derart bereitgestellt sind, dass den individuellen Anforderungen der Patienten hinsichtlich der Zusammensetzung und der Menge der Flüssigkeiten Rechnung getragen werden kann.

Beispielsweise kann einem Patienten nur die parenterale Ernährungslösung aus der einen Kammer oder lediglich die Flüssigkeit zur Deckung des Flüssigkeitsbedarfs aus der anderen Kammer verabreicht werden oder können diese beiden und gegebenenfalls auch weitere Komponenten zur Verabreichung kommen, die unmittelbar vor der Verabreichung durch die regulierbare Flüssigkeitsverbindung gemischt werden können. Wesentlich ist, dass alle Flüssigkeiten in dem Beutel bereitgestellt sind. Ein ganz besonderer Vorteil in der Handhabung besteht dabei darin, dass der Beutel durch den Patienten selbst angelegt werden kann, da nichts mehr zusätzlich aufgezogen oder eingespritzt werden muss. Hierfür ist lediglich eine einfache Schulung des Patienten erforderlich. Durch den damit möglichen Verzicht auf den Pflegedienst ist der Patient flexibel und die Behandlung des Patienten kostengünstiger.

Im Konkreten ist der Innenraum des Beutel erfindungsgemäß in mindestens sechs, vorzugsweise maximal zehn, weiter vorzugsweise in neun, Kammern unterteilt. Hiermit lassen sich durch Bereitstellung unterschiedlicher Flüssigkeiten verschiedene Ernährungskomponenten bereitstellen, so dass eine hinreichende parenterale Ernährung möglich ist. Auch kann mit dieser Kammeranzahl eine hinreichende Anzahl von "Standardkonfigurationen" hergestellt werden, die auf individuelle Rezepturen zurückgehen und - infolge der in separaten Kammern untergebrachten Komponenten - länger gelagert werden können. Untersuchungen zufolge lassen sich somit etwa 70 bis 80% der Individualrezepturen durch etwa 15 bis 20 Standardkonfigurationen abbilden.

Die Flüssigkeit zur Deckung des Flüssigkeitsbedarfs kann Wasser und/oder isotonische Kochsalzlösung aufweisen. Die parenterale Nährlösung umfasst Kohlenhydrate, Aminosäuren, Fette, Vitamine, Spurenelemente und Elektrolyte und kann weiter Mineralstoffe umfassen. Die weiteren der mindestens sechs Kammern können Fettemulsionen, Kohlehydratlösungen, Aminosäurelösungen, Mineralstofflösungen, Spurenelementelösungen und/oder Vitaminlösungen aufweisen.

Die parenterale Ernährungslösung hat zur Deckung des Energiebedarfs des Patienten einen Energiegehalt von mindestens 0,5 kcal/ml, bevorzugt von mindestens 0,8 kcal/ml, besonders bevorzugt von mindestens 1 kcal/ml. Als Eiweißquellen können Aminosäuren und deren Derivate verwendet werden. Als Kohlenhydrate können Poly- und Oligosaccharide eingesetzt werden. Als Fettquellen kommen beispielsweise Soja- sowie Fischöl, MCT (Medium Chain Triglyceride), Olivenöl, Omega-3-Fettsäuren und strukturierte Triglyceride in Betracht.

Zur Herstellung der Flüssigkeitsverbindung können regulierbare Kanäle, insbesondere zumindest teilweise öffenbare und zumindest teilweise wiederverschließbare Kanäle, zwischen den Kammern vorgesehen. Somit können mit einfachen konstruktiven Mitteln ein gezieltes Mischen einzelner parenteraler Komponenten und damit eine Vorbereitung der parenteralen Nahrung erfolgen. Zudem ist eine stabile Lagerung der Ernährungskomponenten möglich, da eine Zusammenführung der Flüssigkeiten erst unmittelbar vor der Verabreichung der parenteralen erfolgt. Bei einer Ausführung mit wiederverschließbaren Kanälen kann eine erneute Zugabe einer Komponente erfolgen, so dass eine bedarfsgerechte, beispielsweise auch eine intermittierende, Zugabe einer Komponente möglich ist.

Im Konkreten kann mittels der Kanäle eine Flüssigkeitsverbindung weiterer Kammern mit der ersten Kammer und/oder der zweiten Kammer herstellbar sein. Die erste und die zweite Kammer können dabei als Entnahmekammern dienen. Ferner ist auch eine gezielte "Vormischung" einzelner Komponenten möglich, so dass beispielsweise vor einer Zusammenführung der Flüssigkeiten in der ersten Kammer und der zweiten Kammer jeweils dort eine der Zusammenführung vorangehende Mischung erfolgen kann. Somit können die erste Kammer und die zweite Kammer auch als "Vorkammer" bezeichnet werden. In diesem Zusammenhang ist es von Vorteil, wenn eine oder mehrere der weiteren Kammern mit der ersten Kammer verbindbar sind und die übrigen der weiteren der mindestens sechs Kammern über deren Kanäle mit der zweiten Kammer verbindbar sind.

Im Wege einer konstruktiv günstigen Ausgestaltung können die regulierbaren Kanäle jeweils durch einen Durchgang in der Wandung einer der Kammern und durch ein Klemmmittel zum Öffnen und Verschließen des Durchgangs, insbesondere einer Klammer, gebildet sein. Neben einer günstigen Konstruktion ist dadurch auch eine einfache Handhabung realisiert, in dem die Klemmmittel ganz oder teilweise von dem Durchgang bildenden Abschnitt des Behältnisses abgenommen werden können, wodurch der Kanal ganz oder teilweise geöffnet wird. Der Öffnungszustand des Kanals lässt sich dabei unmittelbar erkennen, nämlich anhand des Vorhandenseins, des Fehlens oder der Position des Klemmmittels, insbesondere der Klammer. Bei einer Ausgestaltung des Klemmmittels als Klammer kann ein Durchgang auf einfache Weise wieder verschlossen werden, in dem die Klammer "aufgesteckt" wird. Dabei ist denkbar, dass für jeden Durchgang ein Klemmmittel oder eine Klammer verwendet wird. Sind mehrere Ernährungskomponenten nur zusammen verwendbar, ist auch denkbar, dass mehreren Durchgängen lediglich eine Klammer zugeordnet ist.

Zur Ermöglichung einer sicheren Handhabung kann eine durch den Innenraum hindurchragende und von diesem abgetrennte Öffnung zur Aufnahme und/oder zum Einführen des Klemmmittels, insbesondere einer Klammer, vorgesehen sein. Das Klemmmittel oder die Klammer kann durch die Öffnung eingeführt und somit am Durchgang befestigt werden. Auch ein entsprechendes Entfernen lässt sich auf diese Weise realisieren. Durch diese Ausgestaltung ist eine sichere Zuordnung der Klemmmittel am Behältnis realisiert, da diese nicht nach außen hin abragen und das Risiko einer versehentlichen Entfernung der Klemmmittel oder Klammern somit ganz erheblich reduziert wird.

Für eine schnelle und flexible Einsetzbarkeit kann ein mit einer der Kammern koppelbares Überleitsystem zur Verabreichung der parenteralen Nährlösung und/oder der Flüssigkeit zur Deckung des Flüssigkeitsbedarfs an einen Patienten vorgesehen sein. Als Überleitsystem können Infusionssysteme verwendet werden, die über eine Schlauchleitung und entsprechende Anschlüsse verfügen. An einem Ende kann ein Anschlussstück für das Behältnis und an dem anderen Ende ein Anschlussstück für eine intravenöse Applikation aufweisen.

Im Konkreten kann mindestens den weiteren Kammern ein Anschlussstück zum Befüllen oder Entleeren des Kammerinhalts, insbesondere Luer-Lock-Ventilport zugeordnet sein. Somit kann eine individuelle Befüllung oder Entleerung einzelner Kammern erfolgen. Neben einer gezielten Verabreichung des Kammerinhalts aus einer Kammer ist somit auch ein erneutes Befüllen ermöglicht.

Im Wege einer möglichst flexiblen Einsetzbarkeit kann die erste Kammer und/oder die zweite Kammer mindestens einen Zugang, insbesondere ein Nadel-Zuspritzport mit Membran, ein Luer-Lock Zuspritzport, einen Anschluss für ein Infusionsgerät mit Luer-Lock-Verbindung und/oder ein Port für ein Infusionsgerät mit Dorn aufweisen. Neben einer flexiblen Einsetzbarkeit ist somit auch eine hohe Kompatibilität in der Anwendung des Behältnisses gegeben. Außerdem ist auf diese Weise eine Redundanz realisiert, wenn nämlich am Einsatzort einer der Anschlüsse beschädigt wird oder dessen komplementäres - beispielsweise pumpenseitiges - Gegenstück gar nicht erst zur Verfügung steht. Im Hinblick auf die konkrete Anordnung der voranstehend aufgeführten Zugänge und Anschlüsse ist denkbar, dass diese an einer Seite des Behältnisses ausgebildet sind.

Im Hinblick auf eine mögliche Verabreichung mittels Schwerkraft können an der von dem Zugang abgewandten Seite Aufhängepunkte zum Befestigen des Behälters, insbesondere in Form von Durchgängen, vorgesehen sein. Somit ist eine Verabreichung parenteraler Nahrung auch dann gewährleistet, wenn keine aufwendigen medizinischen Einrichtungen, wie beispielsweise Dosierpumpen, zur Verfügung stehen. Durch Ausgestaltung von Aufhängepumpen kann das Behältnis an einem - gegenüber dem Patient erhöhten - Punkt befestigt werden. Dabei ist es, wie vorstehend bereits erläutert, von Vorteil, wenn die Anschlüsse oder Entnahmepunkte der ersten und/oder der zweiten Kammer an einer Seite des Behältnisses angeordnet sind, die den Aufhängepunkten gegenüberliegen. Zur Gewährleistung einer Schwerkraftentnahme von Flüssigkeiten aus jeder der Kammern ist - im Falle einer im Wesentlichen rechteckigen Grundgestalt des Behältnisses - denkbar, dass an jeder Ecke Durchgänge zum Bilden eines Aufnahmepunktes vorgesehen sind.

Die eingangs genannte Aufgabe wird auch durch ein Set mit den Merkmalen des Anspruchs 12 gelöst. Für das erfindungsgemäße Set zur Bereitstellung parenteraler Nahrung ist dabei von besonderer Bedeutung, dass dieses einen erfindungsgemäßen Beutel und ein mit einer Kammer des Beutels koppelbares Überleitsystem zur Verabreichung parenteraler Nahrung an einen Patienten umfasst, wobei dar Beutel und das Überleitsystem in einer geschlossenen Verpackung aufgenommen sind.

Dieses Set erlaubt ein unmittelbares Applizieren der parenteralen Ernährungslösung und/oder der Flüssigkeit zur Deckung des Flüssigkeitsbedarfs, wobei der Beutel, nebst Überleitsystem oder Spiralleitung in der Verpackung enthalten sind. Dabei ist von Vorteil, wenn Beutel und Überleitsystem steril in der Verpackung aufgenommen sind. Hierzu kann die Verpackung, die vorzugsweise als Beutel ausgebildet ist, aus einer Folie bestehen, die eine Sterilisation Mehrkammerbeutels, ermöglicht. Auch ist für eine sofortige Einsetzbarkeit des Sets von Vorteil, wenn zumindest die parenterale Nährlösung und die Flüssigkeit zur Deckung des Flüssigkeitsbedarfs bereits im Beutel enthalten sind, so dass der Beutel und damit das Set insgesamt gebrauchsfertig sind.

Als Überleitsystem oder Spiralleitung können Infusionssysteme verwendet werden, die über eine Schlauchleitung mit entsprechendem Anschlussstücken verfügen. An einem Ende kann ein Anschlussstück für den Beutel und an dem anderen Ende ein Anschlussstück für eine intravenöse Applikation vorgesehen sein. Für eine möglichst einfache Entnahme des Beutels und des Überleitsystems aus der Verpackung kann diese als Folienbeutel ausgeführt sein. Der Folienbeutel kann aus einer flexiblen Kunststofffolie ausgebildet sein, die an einer Seite gefaltet und an den weiteren Seiten durch eine Schweißnaht verschlossen ist. Durch Ausgestaltung eines Öffnungsbereichs ist eine einfache Entnahme des Beutels und des Überleitsystems möglich, und zwar weitestgehend ohne diese Komponenten zu verunreinigen. Der öffenbare Bereich kann als auftrennbare Naht, insbesondere als ein auftrennbarer Nahtabschnitt der Schweißnaht, oder als Perforation ausgeführt sein, die ein einfaches manuelles Öffnen der Verpackung ermöglicht.

Es gibt nun verschiedene Möglichkeiten, die Lehre der vorliegenden Erfindung in vorteilhafter Weise auszugestalten und weiterzubilden. Die Erfindung wird dabei durch die Ansprüche definiert. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Ansicht ein erstes Ausführungsbeispiel eines erfindungsgemäßen Behältnisses zur Bereitstellung parenteraler Nahrung,
- Fig. 2: in einer schematischen Ansicht ein Ausführungsbeispiel eines erfindungsgemäßen Sets zur Bereitstellung parenteraler Nahrung, umfassend ein erfindungsgemäßes Behältnis und
- Fig. 3: in einer schematischen Ansicht ein zweites Ausführungsbeispiel eines erfindungsgemäßen Behältnisses.

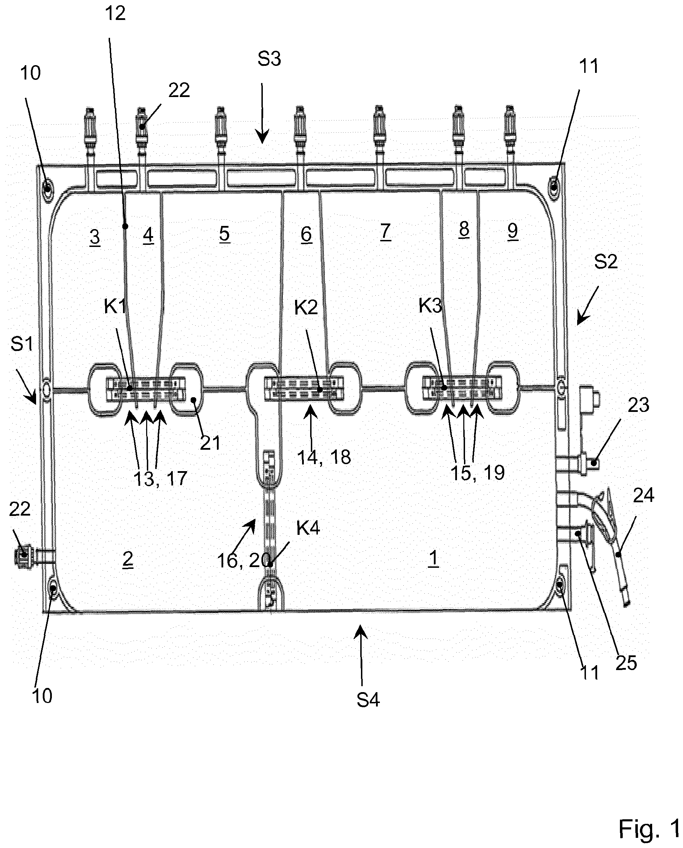

Fig. 1 zeigt ein Ausführungsbeispiel eines erfindungsgemäßen Behältnisses zur Bereitstellung parenteraler Nahrung. Das Behältnis ist als Beutel, und zwar als polymerer Mehrkammerbeutel, ausgebildet. Der Mehrkammerbeutel weist neun voneinander abgetrennte Kammern 1 bis 9 auf.

Der Beutel ist aus einer umgeschlagenen flexiblen Kunststofffolie gebildet, die an der ersten Seite S1 und der zweiten Seite S2 mit einer doppelten Schweißnaht und an der - in Fig. 1 oberen - dritten Seite S3 mit einer einfachen Schweißnaht nach außen hin verschlossen ist. Die vierte Seite S4 benötigt keine Schweißnaht, da die Folie hier umgeschlagen wurde und somit nahtlos verschlossen ist.

Zur Befestigung des Beutels sind an der ersten Seite S1 Befestigungspunkte 10 in Form von Durchgängen 10 ausgebildet. An der zweiten Seite 2 sind Befestigungspunkte 11 in Form von Durchgängen 11 ausgebildet. Somit kann eine Flüssigkeitsentnahme aufgrund Schwerkrafteinwirkung erfolgen, wobei der Beutel derart aufzuhängen ist, dass die Entnahmestelle zum Erdboden hin, einfacher gesagt "nach unten", weist.

Die Kammern 1 bis 9 sind durch feste Kammerwandungen 12 - zur Übersicht lediglich einmal mit Bezugszeichen versehen - voneinander abgetrennt. Die Kammern 3, 4 und 5 weisen jeweils einen regulierbaren Kanal 13 auf, mit dem eine Flüssigkeitsverbindung zur zweiten Kammer 2 hergestellt werden kann. Die Kammer 6 weist einen Kanal 14 und die Kammern 7, 8 und 9 weisen jeweils einen regulierbaren Kanal 15 auf. Damit können die Kammern 6, 7, 8, 9 im Wege einer Flüssigkeitsverbindung mit der ersten Kammer 1 verbunden werden.

Die erste Kammer 1 und die zweite Kammer 2 können durch einen Kanal 16 zur Herstellung einer Flüssigkeitsverbindung miteinander gekoppelt werden. Die regulierbaren Kanäle 13, 14, 15, 16 werden jeweils durch einen Durchgang 17, 18, 19, 20 und durch Klemmmittel K1, K2, K3, K4 zum Öffnen und Verschließen des Durchgangs 17, 18, 19, 20 gebildet. Auch ein teilweises Öffnen oder ein teilweises Verschließen ist möglich. Die Klemmmittel K1, K2, K3, K4 sind als Klammern K1, K2, K3, K4 ausgebildet. Die Klemmmittel oder Klammern K1, K2, K3, K4 können ganz oder teilweise von Durchgängen entfernt werden, so dass eine Regulierung der aus einer Kammer entnehmbaren Flüssigkeitsmenge erfolgen kann. Auch ein erneutes Anbringen der Klemmmittel oder Klammern K1, K2, K3, K4 ist denkbar, so dass auch ein Wiederverschließen und ggf. erneutes Befüllen einer Kammer möglich ist.

Zur Aufnahme oder zum Einführen der Klammern K1, K2, K3, K4 sind durch den Innenraum des Beutels hindurchragende und von diesem abgetrennte Öffnungen 21 - zur Übersichtlichkeit lediglich einmal mit Bezugszeichen gekennzeichnet - vorgesehen. Auf diese Weise sind die Klammern K1, K2, K3, K4 im Inneren des Beutels angeordnet und ragen zur Seite hin nicht vom Beutel ab, so dass das Risiko eines versehentlichen Lösens der Klammern minimiert ist. Gleichzeitig kann durch Vorhandensein, Fehlen oder der Position der Klammern K1, K2, K3, K4 unmittelbar erkannt werden, ob die Durchgänge 17, 18, 19, 20 geöffnet oder geschlossen sind oder teilweise geöffnet oder geschlossen sind. Die Klemmmittel oder Klammern K1, K2, K3, K4 können auch einzeln geöffnet werden, so dass sich entsprechend der jeweiligen Flüssigkeiten in den Kammern 1 bis 9 individuelle Mischungen verwirklichen lassen. Dadurch ist ein individuelles Eingehen auf die Stoffwechsellage eines Patienten möglich.

In den Kammern 2 bis 9 ist jeweils ein Anschlussstück 22 zum Befüllen oder Entleeren des Kammerinhalts zugeordnet. Das Anschlussstück 22 ist im vorliegenden Ausführungsbeispiel als Luer-Lock-Ventilport 22 ausgebildet. Damit ist nicht nur eine gezielte Entnahme oder Applikation einer der Flüssigkeiten möglich, sondern auch ein Auffüllen oder Nachfüllen einer benötigten Flüssigkeit.

Die erste Kammer 1 weist ein Nadel-Zuspritzport mit Membran 23, ein Luer-Lock-Zuspritzport oder Anschluss für ein Infusionsgerät mit Luer-Lock-Verbindung 24 und ein Port für ein Infusionsgerät mit Dorn 25 auf. Hierdurch ist eine flexible Anschlussmöglichkeit des Beutels 1 zur Bereitstellung parenteraler Nahrung ermöglicht. Auch ist damit eine redundante Anschlussmöglichkeit verwirklicht, wenn am Einsatzort das komplementäre Gegenstück zu einem der am Beutel ausgebildeten Anschlüsse defekt oder gar nicht erst vorhanden ist.

Die Kanäle 14 und 16 weisen jeweils eine Klammer K2, K4 auf. Die Kanäle 13 und 15 der Kammern 3, 4, 5 und 7, 8, 9 weisen jeweils eine Klammer K1, K3 für mehrere Kanäle auf.

Im vorliegenden Ausführungsbeispiel weist der Beutel eine Länge von 600 mm und eine Breite von 250 mm auf. Die Kammern 1 bis 9 sind in ihrer Größe unterschiedlich, nämlich gestuft, dimensioniert. Damit können verschiedene Flüssigkeiten in der jeweils benötigten Menge im Beutel gespeichert werden. Zudem weist die erste Kammer 1, die zur Aufnahme der parenteralen Nährlösung dient, ein größeres Volumen auf als die zweite Kammer 2, die zur Aufnahme einer Flüssigkeit zur Deckung des Flüssigkeitsbedarfs dient. Die erste Kammer 1 weist ein Fassungsvermögen von 1.000 ml auf, während die zweite Kammer 2 ein Volumen von 600 ml aufnehmen kann. Die Kammern 5 und 7 weisen ein Fassungsvermögen von jeweils 100 ml auf, wohingegen die Kammern 4, 6 und 8 ein Fassungsvermögen von je 15 ml aufweisen. Die Kammern 3 und 9 können jeweils ein Volumen von 80 ml aufnehmen.

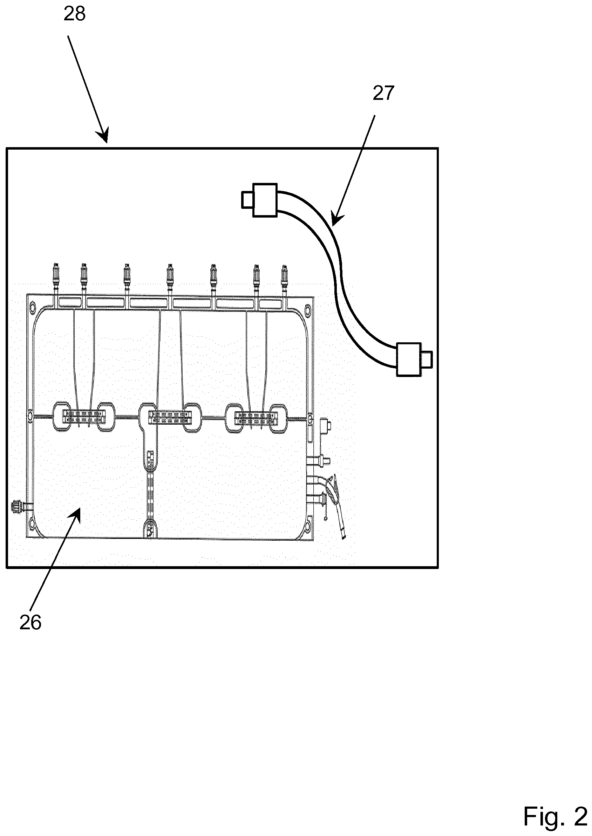

Fig. 2 zeigt ein Ausführungsbeispiel eines erfindungsgemäßen Sets zur Bereitstellung parenteraler Nahrung. Das Set umfasst ein erfindungsgemäßes Behältnis 26 mit darin enthaltenen parenteralen Flüssigkeiten, nämlich zumindest einer parenteralen Nährlösung und einer Flüssigkeit zur Deckung des Flüssigkeitsbedarfs. Zudem ist ein mit einer der Kammern des Behältnisses 26 koppelbares - hier nur schematisch dargestelltes - Überleitsystem 27 zur Verabreichung parenteraler Nahrung an einen Patienten im Set enthalten. Als Überleitsystem dient ein Infusionssystem, das über eine Schlauchleitung und entsprechende Anschlüsse verfügt. An einem Ende ist ein Anschlussstück zum Anschluss an das Behältnis und an dem anderen Ende ein Anschlussstück für eine intravenöse Applikation ausgebildet.

Das Behältnis 26 und das Überleitsystem 27 sind in einer geschlossenen und sterilen Verpackung 28 aufgenommen. Die Verpackung 28 ist als Beutel aus übereinanderliegenden Kunststofffolien gebildet, die durch eine - hier nicht dargestellte - umlaufende Schweißnaht miteinander verschweißt sind. Zumindest ein Teil der - nicht dargestellten - Schweißnaht ist als aufreißbare oder auftrennbare Schweißnaht, die auch als Peelnaht bezeichnet werden kann, ausgebildet. Daher ist es möglich, die sterile Verpackung 28 auf einfache Art und Weise zu öffnen, um die Bestandteile des Sets zu entnehmen. Dadurch, dass das Set den Beutel 26 und das Überleitungssystem 27 in sterilem und anwendungsfertigen Zustand in der Verpackung 28 bereitstellt, kann eine unmittelbare und zügige Verabreichung parenteraler Nahrung erfolgen, da die Verpackung 28 lediglich geöffnet werden muss und danach die parenterale Nahrung appliziert werden kann.

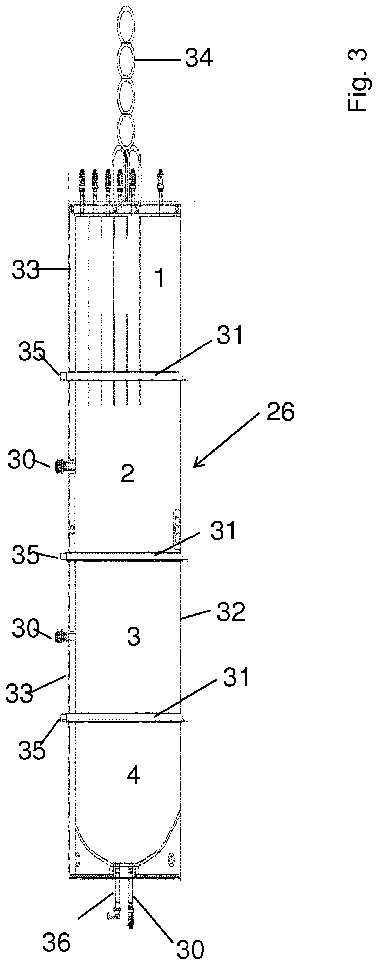

Fig. 3 zeigt ein weiteres Ausführungsbeispiel eines erfindungsgemäßen Behältnisses (26), wobei das Behältnis (26) aus einer gefalteten und verschweißten Folienbahn (32) besteht. Zum Halten bzw. zur Aufnahme des Beutels (26) ist eine Art Rahmen (33) vorgesehen, der als Aufnahme bzw. Aufhängung für den Behälter (26) dient. Der Rahmen (33) verfügt wiederum über eine Aufhängeeinrichtung (34), die aus einer Anordnung von Ringösen oder dergleichen gebildet sein kann.

Für den in Fig. 3 gezeigten Behälter (26) ist wesentlich, dass dieser durch insgesamt drei Klemmschienen (31) in vier Kammern 1, 2, 3, 4 unterteilt ist, wobei die Klemmschiene über eine eingelegte bzw. integrierte Klemmlitze (35) verfügt. Wird die Klemmlitze (35) auf der Klemmschiene (31) ganz oder teilweise herausgezogen, wird eine Strömungsverbindung zwischen den Kammern 1, 2, 3, 4 hergestellt und lassen sich die im Behälter (26), im Konkreten in den dortigen Kammern 1, 2, 3, 4, befindlichen Fluide untereinander vermischen.

Bei dem in Fig. 3 gezeigten Ausführungsbeispiel ist eine Regulierung im Wege einer Strömungsverbindung zwischen den Kammern 1, 2, 3, 4 nicht möglich, so dass eine insgesamte Vermischung zwischen den Fluiden nach Entfernen der Klemmlitze (35) erfolgt. Will man den Inhalt einer Kammer nicht oder nur teilweise für die Mischung verwenden, kann man die Kammer ganz oder teilweise über ein Ventile (30) entleeren.

Nach dem Vermischen der Inhalte der Kammer 1, 2, 3, 4 lässt sich die Mischung über das Auslassventil (36) verabreichen.

Hinsichtlich weiterer vorteilhafter Ausgestaltungen des erfindungsgemäßen Behältnisses sowie des erfindungsgemäßen Sets wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele des erfindungsgemäßen Behältnisses und des erfindungsgemäßen Sets lediglich zur Erörterung der beanspruchten Lehre dienen, diese jedoch nicht auf die Ausführungsbeispiele einschränken.

### Bezugszeichenliste

- 1: erste Kammer
- 2: zweite Kammer
- 3, 4, 5, 6, 7, 8, 9: Kammer
- 10, 11: Aufhängepunkt, Durchgang
- 12: Wandung
- 13, 14, 15, 16: Kanal
- 17,18,19,20: Durchgang
- 21: Öffnung
- 22: Anschlussstück, Luer-Lock-Ventilport
- 23: Nadel-Zuspritzport
- 24: Luer-Lock-Zuspritzport
- 25: Port für Infusionsgerät mit Dorn
- 26: Behälter, Beutel, polymerer Mehrkammerbeutel
- 27: Überleitsystem
- 28: Verpackung
- 30: Ventile
- 31: Klemmschiene
- 32: Folienbahn
- 33: Rahmen/Teilrahmen
- 34: Aufhängeeinrichtung
- 35: Klemmlitze
- 36: Auslassventil
- K1, K2, K3, K4: Klemmmittel, Klammer
- S1, S2, S3, S4: Seite

## Patentansprüche

1. Beutel (26) zur Bereitstellung parenteraler Nahrung entsprechend der individuellen Bedürfnisse des Patienten, mit einem in getrennte Kammern (1, 2, 3, 4) unterteilten Innenraum, wobei die Kammern (1, 2, 3, 4) zur Aufnahme von Flüssigkeiten zur parenteralen Ernährung dienen, wobei im Innenraum mindestens zwei Kammern (1, 2, 3, 4) ausgebildet sind, zwischen denen eine Flüssigkeitsverbindung herstellbar ist, wobei die Kammern (1, 2, 3, 4) durch sich quer zur Längsrichtung und über die gesamte Breite des Beutels (26) erstreckende Klemmschienen (31) gebildet und gegeneinander abgegrenzt sind, wobei in den Klemmschienen (31) Litzen (35) angeordnet sind, die in der Klemmschiene (31) klemmen und die ganz oder teilweise aus der Klemmschiene (31) herausziehbar sind, wodurch die Flüssigkeitsverbindung zwischen den Kammern (1, 2, 3, 4) herstellbar ist, und wobei der Innenraum des Beutels in mindestens sechs und vorzugsweise maximal zehn, Kammern (1, 2, 3, 4) unterteilt ist, wobei mindestens zwei Kammern (1, 2, 3, 4) entsprechend der individuellen Stoffwechsellage eines Patienten unterschiedliche Ernährungskomponenten enthalten, die nach Herstellung der Flüssigkeitsverbindung zwischen den Kammern (1, 2, 3, 4) und vor Verabreichung an den Patienten zu einer parenteralen Nahrung vermischbar sind, wobei mindestens eine erste Kammer (1) eine parenterale Nährlösung mit einem ersten Energiegehalt enthält und mindestens eine zweite Kammer (2) eine Flüssigkeit zur Deckung des Flüssigkeitsbedarfs mit einem zweiten Energiegehalt enthält, der kleiner als der erste Energiegehalt der parenteralen Nährlösung ist, wobei die parenterale Nährlösung Kohlenhydrate, Aminosäuren, Fette, Vitamine, Spurenelemente und Elektrolyte umfasst.

2. Beutel nach Anspruch 1, **dadurch gekennzeichnet, dass** die Flüssigkeitsverbindung zwischen den Kammern (1, 2, 3, 4, 5, 6, 7, 8, 9) regulierbar ist.

3. Beutel nach Anspruch 1 oder 2, **dadurch gekennzeichnet, dass** die Kammern (1, 2, 3, 4, 5, 6, 7, 8, 9) vorzugsweise über ein Ventil (30) nach außen öffenbar und ggf. insgesamt oder teilweise, vorzugsweise dosiert, nach außen entleerbar sind.

4. Beutel nach Anspruch 1 bis 3, **dadurch gekennzeichnet, dass** die erste Kammer (1) ein größeres Volumen als die zweite Kammer (2) aufweist.

5. Beutel nach Anspruch 1 bis 4, **dadurch gekennzeichnet, dass** weitere Kammern (7, 8, 9) im Innenraum vorgesehen sind, die zur bedarfsgerechten Bereitstellung von Flüssigkeiten im Volumen unterschiedlich, insbesondere gestuft, dimensioniert sind und/oder dass der Innenraum in neun, Kammern (1, 2, 3, 4, 5, 6, 7, 8, 9) unterteilt ist.

6. Beutel nach einem der Ansprüche 1 bis 5, **dadurch gekennzeichnet, dass** zur Herstellung der Flüssigkeitsverbindung regulierbare Kanäle (14, 15, 16, 20), insbesondere zumindest teilweise öffenbare und zumindest teilweise wiederverschließbare Kanäle (13, 14, 15, 16), zwischen den Kammern (1, 2, 3, 4, 5, 6, 7, 8, 9) vorgesehen sind und/oder dass mittels der Kanäle (13, 14, 15, 16) eine Flüssigkeitsverbindung weiterer Kammern (3, 4, 5, 6, 7, 8, 9) mit der ersten Kammer (1) und/oder der zweiten Kammer (2) herstellbar ist.

7. Beutel nach Anspruch 6, **dadurch gekennzeichnet, dass** die regulierbaren Kanäle (13, 14, 15, 16) jeweils durch einen Durchgang (17, 18, 19, 20) in einer Wandung (12) einer der Kammern (1, 2, 3, 4, 5, 6, 7, 8, 9) und durch ein Klemmmittel (K1, K2, K3, K4) zum Öffnen und Verschließen des Durchgangs (17, 18, 19, 20), insbesondere einer Klammer (K1, K2, K3, K4), gebildet sind.

8. Beutel nach Anspruch 7, **dadurch gekennzeichnet, dass** eine durch den Innenraum hindurch ragende und von diesem abgetrennte Öffnung (21) zur Aufnahme und/oder zum Einführen des Klemmmittels (K1, K2, K3, K4), insbesondere der Klammer (K1, K2, K3, K4), vorgesehen ist.

9. Beutel nach einem der Ansprüche 1 bis 8, **dadurch gekennzeichnet, dass** ein mit einer der Kammern (1, 2, 3, 4, 5, 6, 7, 8, 9) koppelbares Überleitsystem (27) zur Verabreichung der parenteralen Nährlösung und/oder der Flüssigkeit zur Deckung des Flüssigkeitsbedarfs an einen Patienten vorgesehen ist und/oder dass mindestens den weiteren Kammern (3, 4, 5, 6, 7, 8, 9) ein Anschlusstück (22) zum Befüllen oder Entleeren des Kammerinhalts, insbesondere ein Luer-Lock-Ventilport (22), zugeordnet ist.

10. Beutel nach einem der Ansprüche 1 bis 9, **dadurch gekennzeichnet, dass** die erste Kammer (1) und/oder die zweite Kammer (2) mindestens einen Zugang (22, 23), insbesondere einen Nadel-Zuspritzport (23) mit Membran, ein Luer-Lock Zuspritzport (24), einen Anschluss für ein Infusionsgerät mit Luer-Lock-Verbindung und/oder ein Port (25) für ein Infusionsgerät mit Dorn aufweisen/aufweist.

11. Beutel nach Anspruch 10, **dadurch gekennzeichnet, dass** an der von dem Zugang (22, 23, 24, 25) abgewandten Seite Aufhängepunkte (10, 11) zum Befestigen des Beutels, insbesondere in Form von Durchgängen (10, 11), vorgesehen sind.

12. Set zur Bereitstellung parenteraler Nahrung umfassend ein Beutel (26) nach einem der Ansprüche 1 bis 9 und ein mit einer Kammer des Beutels (26) koppelbares Überleitsystem (27) zur Verabreichung parenteraler Nahrung an einen Patienten, wobei der Beutel (26) und das Überleitsystem (27) in einer geschlossenen Verpackung (28) aufgenommen sind.

## Claims

1. Bag (26) for providing parenteral nutrition in accordance with the individual requirements of the patient, having an inner space which is subdivided into separate chambers (1, 2, 3, 4), wherein the chambers (1, 2, 3, 4) serve to receive fluids for parenteral nutrition, wherein in the inner space there are formed at least two chambers (1, 2, 3, 4), between which a fluid connection can be produced, wherein the chambers (1, 2, 3, 4) are formed by clamping rails (31) which extend transversely relative to the longitudinal direction and over the entire width of the bag (26) and which are delimited with respect to each other, wherein in the clamping rails (31) there are arranged strands (35) which clamp in the clamping rail (31) and which can be pulled completely or partially out of the clamping rail (31), whereby the fluid connection between the chambers (1, 2, 3, 4) can be produced, and wherein the inner space of the bag is subdivided into a minimum of six and preferably a maximum of ten chambers (1, 2, 3, 4), wherein at least two chambers (1, 2, 3, 4) in accordance with the individual metabolic state of a patient contain different nutritional components, which after production of the fluid connection between the chambers (1, 2, 3, 4) and before administration to the patient can be mixed to form a parenteral nutrition, wherein at least a first chamber (1) contains a parenteral nutrition solution with a first energy content and at least a second chamber (2) contains a fluid to cover the fluid requirement with a second energy content which is smaller than the first energy content of the parenteral nutrition solution, wherein the parenteral nutrition solution comprises carbohydrates, amino acids, fats, vitamins, trace elements and electrolytes.

2. Bag according to claim 1, **characterised in that** the fluid connection between the chambers (1, 2, 3, 4, 5, 6, 7, 8, 9) can be controlled.

3. Bag according to claim 1 or claim 2, **characterised in that** the chambers (1, 2, 3, 4, 5, 6, 7, 8, 9) can preferably be opened outwards by means of a valve (30) and where applicable completely or partially, preferably in a metered manner, emptied outwards.

4. Bag according to claims 1 to 3, **characterised in that** the first chamber (1) has a larger volume than the second chamber (2) .

5. Bag according to claims 1 to 4, **characterised in that** there are provided in the inner space additional chambers (7, 8, 9) which in order to provide fluids in accordance with requirements are sized differently in terms of volume, in particular in a stepped manner, and/or **in that** the inner space is subdivided into nine chambers (1, 2, 3, 4, 5, 6, 7, 8, 9).

6. Bag according to any one of claims 1 to 5, **characterised in that** in order to produce the fluid connection controllable channels (14, 15, 16, 20), in particular channels (13, 14, 15, 16) which can be at least partially opened and at least partially closed again, are provided between the chambers (1, 2, 3, 4, 5, 6, 7, 8, 9) and/or **in that** by means of the channels (13, 14, 15, 16) a fluid connection of additional chambers (3, 4, 5, 6, 7, 8, 9) to the first chamber (1) and/or the second chamber (2) can be produced.

7. Bag according to claim 6, **characterised in that** the controllable channels (13, 14, 15, 16) are each formed by means of a passage (17, 18, 19, 20) in a wall (12) of one of the chambers (1, 2, 3, 4, 5, 6, 7, 8, 9) and by a clamping means (K1, K2, K3, K4) for opening and closing the passage (17, 18, 19, 20), in particular a clamp (K1, K2, K3, K4).

8. Bag according to claim 7, **characterised in that** an opening (21) which protrudes through the inner space and which is separated therefrom is provided for receiving and/or for introducing the clamping means (K1, K2, K3, K4), in particular the clamp (K1, K2, K3, K4).

9. Bag according to any one of claims 1 to 8, **characterised in that** a transfer system (27) which can be coupled to one of the chambers (1, 2, 3, 4, 5, 6, 7, 8, 9) in order to administer to a patient the parenteral nutrition solution and/or the fluid to cover the fluid requirement is provided and/or **in that** a connection piece (22) for filling or emptying the chamber content, in particular a Luer lock valve port (22), is associated with at least the additional chambers (3, 4, 5, 6, 7, 8, 9).

10. Bag according to any one of claims 1 to 9, **characterised in that** the first chamber (1) and/or the second chamber (2) has/have at least one access (22, 23), in particular a needle injection port (23) having a diaphragm, a Luer lock injection port (24), a connection for an infusion device having a Luer lock connection and/or a port (25) for an infusion device having a spike-like member.

11. Bag according to claim 10, **characterised in that** at the side facing away from the access (22, 23, 24, 25) suspension locations (10, 11) for securing the bag, in particular in the form of channels (10, 11), are provided.

12. Set for providing parenteral nutrition comprising a bag (26) according to any one of claims 1 to 9 and a transfer system (27) which can be coupled to a chamber of the bag (26) for administering parenteral nutrition to a patient, wherein the bag (26) and the transfer system (27) are received in a closed packaging (28).

## Revendications

1. Sachet (26) pour la mise à disposition d'une nutrition parentérale en fonction des besoins individuels du patient, avec un espace interne divisé en chambres séparées (1, 2, 3, 4), les chambres (1, 2, 3, 4) permettant de loger des liquides pour la nutrition parentérale, moyennant quoi, dans l'espace intérieur, sont réalisées au moins deux chambres (1, 2, 3, 4), entre lesquelles une liaison de liquide peut être établie, les chambres (1, 2, 3, 4) étant constituées par rails de serrage (31) s'étendant transversalement par rapport à la direction longitudinale ou sur toute la largeur du sachet (26) et étant délimitées les unes par rapport aux autres, moyennant quoi, dans les rails de serrage (31), sont disposés des torons (35) qui se serrant dans le rail de serrage (31) et peut être retirés entièrement ou partiellement hors du rail de serrage (31), ce qui permet d'établir la liaison de liquide entre les chambres (1, 2, 3, 4) et
l'espace intérieur du sachet étant divisé en au moins six et de préférence au maximum dix chambres (1, 2, 34), au moins deux chambres (1, 2, 3, 4) contenant des composants nutritionnels différents selon l'état du métabolisme individuel d'un patient, qui peuvent être mélangés, après l'établissement de la liaison de liquide entre les chambres (1, 2, 3, 4) et avant l'administration au patient pour une nutrition parentérale, au moins une première chambre (1) contenant une solution nutritive parentérale avec une première teneur énergétique et au moins une deuxième chambre (2) contenant un liquide permettant de couvrir les besoins en liquide avec une deuxième teneur énergétique, qui est inférieure à la première teneur énergétique de la solution nutritive parentérale, la solution nutritive parentérale comprenant des hydrates de carbone, des acides aminés, des graisses, des vitamines, des oligo-éléments et des électrolytes.

2. Sachet selon la revendication 1, **caractérisé en ce que** la liaison de liquide entre les chambres (1, 2, 3, 4, 5, 6, 7, 8, 9) peut être régulée.

3. Sachet selon la revendication 1 ou 2, **caractérisé en ce que** les chambres (1, 2, 3, 4, 5, 6, 7, 8, 9) peuvent être ouvertes vers l'extérieur et, le cas échéant, être vidées vers l'extérieur, entièrement ou partiellement, de préférence de manière dosée, de préférence par l'intermédiaire d'une soupape (30).

4. Sachet selon la revendication 1 à 3, **caractérisé en ce que** la première chambre (1) comprend un volume plus grand que la deuxième chambre (2).

5. Sachet selon la revendication 1 à 4, **caractérisé en ce que** d'autres chambres (7, 8, 9) sont prévues dans l'espace intérieur, qui sont dimensionnées en volume différemment, plus particulièrement de manière échelonnée, pour la mise à disposition de liquides selon les besoins et/ou **en ce que** l'espace intérieur est divisé en neuf chambres (1, 2, 3, 4, 5, 6, 7, 8, 9).

6. Sachet selon l'une des revendications 1 à 5, **caractérisé en ce que**, pour l'établissement de la liaison de liquide, des canaux régulables (14, 15, 16, 20), plus particulièrement des canaux (13, 14, 15, 16) au moins partiellement ouvrables et au moins partiellement refermables, sont prévus et/ou **en ce que**, au moyen des canaux (13, 14, 15, 16), une liaison de liquide d'autres chambres (3, 4, 5, 6, 7, 8, 9) avec la première chambre (1) et/ou la deuxième chambre (2) peut être établie.

7. Sachet selon la revendication 6, **caractérisé en ce que** les canaux régulables (13, 14, 15, 16) sont constitués chacun d'un passage (17, 18, 19, 20) dans une paroi (12) d'une des chambres (1, 2, 3, 4, 5, 6, 7, 8, 9) et d'un moyen de serrage (K1, K2, K3, K4) pour l'ouverture et la fermeture du passage (17, 18, 19, 20), plus particulièrement d'une agrafe (K1, K2, K3, K4).

8. Sachet selon la revendication 7, **caractérisé en ce qu'**une ouverture (21) dépassant à travers l'espace intérieur et séparée de celui-ci, est prévue pour le logement et/ou l'introduction du moyen de serrage (K1, K2, K3, K4), plus particulièrement de l'agrafe (K1, K2, K3, K4).

9. Sachet selon l'une des revendications 1 à 8, **caractérisé en ce qu'**un système de transfert (27), pouvant être couplé avec une des chambres (1, 2, 3, 4, 5, 6, 7, 8, 9), est prévu pour l'administration de la solution nutritive parentérale et/ou du liquide permettant de couvrir les besoins en liquide à un patient et/ou **en ce qu'**au moins aux autres chambres (3, 4, 5, 6, 7, 8, 9) correspond un raccord (22) pour le remplissage ou le vidage du contenu de la chambre, plus particulièrement un orifice à soupape Luer-Lock (22).

10. Sachet selon l'une des revendications 1 à 9, **caractérisé en ce que** la première chambre (1) et/ou la deuxième chambre (2) comprend au moins un accès (22, 23), plus particulièrement un orifice d'injection par aiguille (23) avec une membrane, un orifice d'injection Luer-Lock (24), un raccord pour un appareil de perfusion avec liaison Luer-Lock et/ou un orifice (25) pour un appareil de perfusion avec mandrin.

11. Sachet selon la revendication 10, **caractérisé en ce que**, sur le côté opposé à l'accès (22, 23, 24, 25), sont prévus des points d'accrochage (10, 11) pour la fixation du sachet, plus particulièrement sous la forme de passages (10, 11).

12. Ensemble pour la mise à disposition d'une nutrition parentérale comprenant un sachet (26) selon l'une des revendications 1 à 9 et un système de transfert (27), pouvant être couplé avec une chambre du sachet (26), pour l'administration d'une nutrition parentérale à un patient, le sachet (26) et le système de transfert (27) étant logés dans un emballage fermé (28).
